Europäisches Patentamt

European Patent Office

Office européen des brevets

(11) Publication number: **0 123 528**
**B1**

(12) **EUROPEAN PATENT SPECIFICATION**

(45) Date of publication of patent specification: **09.09.87**

(21) Application number: **84302686.5**

(22) Date of filing: **19.04.84**

(51) Int. Cl.⁴: **A 61 K 7/06**

(54) **Pharmaceutical composition for the treatment of hair loss.**

(30) Priority: **19.04.83 GB 8310543**

(43) Date of publication of application:
**31.10.84 Bulletin 84/44**

(45) Publication of the grant of the patent:
**09.09.87 Bulletin 87/37**

(84) Designated Contracting States:
**AT BE CH DE FR GB IT LI LU NL SE**

(56) References cited:
**EP-A-0 033 164**
**FR-A-2 343 474**
**FR-M- 6 434**
**GB-A- 859 546**
**GB-A- 874 368**

(73) Proprietor: **Mortimer, Christopher Harry, Dr.**
**The Endocrine and Dermatology Centre 140**
**Harley street**
**London W1N 1AH (GB)**

(72) Inventor: **Mortimer, Christopher Harry, Dr.**
**The Endocrine and Dermatology Centre 140**
**Harley street**
**London W1N 1AH (GB)**

(74) Representative: **Baverstock, Michael George**
**Douglas et al**
**BOULT, WADE & TENNANT 27 Furnival Street**
**London, EC4A 1PQ (GB)**

The file contains technical information
submitted after the application was filed and
not included in this specification

Courier Press, Leamington Spa, England.

EP 0 123 528 B1

**0 123 528**

## Description

The present invention relates to a pharmaceutical composition for preventing or arresting scalp hair loss and/or promoting scalp hair growth and in particular to a composition for topical application.

The present invention provides a pharmaceutical composition for preventing or arresting scalp hair loss and/or promoting scalp hair growth comprising an anti-androgen, an oestrogen and a thyroid hormone. The compositions according to the invention may in general be used either by male or female patients.

GB—A—859,546 describes the topical use of thyroid hormones, in alopecia (not specified) in combination with oestrogens and androgens, (but not with a non-oestrogenic anti-androgen). Clinical studies were not reported.

GB—A—874,368 describes the topical use of an oestrogen and a non-oestrogenic anti-androgen (progesterone) but not in combination with a thyroid hormone as an anti-seborrhoeic preparation. It allows for the possible inclusion of the amino acid, methionine, together with a vasodilator. No suggestion of possible use to promote new scalp hair growth is made.

FR—A—2,343,474 describes the effect of topical cyproterone acetate alone on sebum secretion from the scalp in man. It is claimed that the preparation might control skin conditions such as seborrhoea, acne vulgaris and hirsuitism. No suggestion of its use to promote new scalp hair growth is made.

EP—A—0,033,164 describes the use of oestrogens and progestogens in tablet form but not in combination with a thyroid hormone, in the treatment of hair loss in women.

FR—M—6,434 describes the use of L-thyroxine with Vitamin A and an oestrogen for topical application in skin disorders to include alopecia (not specified) and acne (presumably acne vulgaris). The combination of a thyroid hormone with an oestrogen and a non-oestrogenic anti-androgen is not described.

According to the present invention there is provided a pharmaceutical composition for promoting scalp hair growth comprising in combination an oestrogen in a concentration above 0.01% by weight to the maximum solubility of same in its solvent, a non-oestrogenic anti-androgen in a concentration above 0.01% by weight to the maximum solubility of same in its solvent and an amount of thyroid hormone selected from 3,3⁻,5-triiodo-L-thyronine and the pharmaceutically acceptable derivatives or precursors thereof, sufficient to provide a concentration of at least 0.66 µg/ml to the maximum solubility of same in its solvent.

An antiandrogen is herein defined as an agent which suppresses the secretion of androgens from either the adrenal glands or testes or ovaries either by suppressing pituitary gonadotrophin or ACTH/secretion or reducing androgen secretion directly from the adrenal glands, ovaries or testes themselves, or is effective locally within the tissues by either displacing androgen from its tissure receptor or competing with its action, or which has an opposite action to androgens (e.g. oestrogen) or impairs the conversion of an androgen substrate to its biologically more active form as testosterone or dihydrotestosterone (e.g. 5 alpha reductase inhibitors such as progesterone), or inhibits in any other way the synthesis and/or the secretion and/or the action of androgens.

The composition according to this invention includes at least one oestrogen, e.g. oestradiol or a pharmaceutically suitable derivative thereof such as oestradiol benzoate, or ethinyloestradiol, in combination with at least one non-oestrogenic anti-androgen such as a progesterone-like compound e.g. medroxyprogesterone acetate or a similarly pharmaceutically acceptable derivative of medroxyprogesterone. Another suitable non-oestrogenic anti-androgen for use in the compositions according to this invention would be cyproterone acetate or another pharmaceutically suitable derivative of cyproterone. Although cyproterone acetate and medroxyprogesterone acetate are the preferred antiandrogens for use in the compositions according to the invention, other antiandrogens having a progesterone-like activity may suitably be employed. These include allyloestrenol, gestronol (e.g. as the hexanoate), dydrogesterone, norgestrel, levonorgestrel, norethisterone, hydroxyprogesterone (e.g. as the hexanoate), progesterone itself, cimetidine or azelaic acid.

The thyroid hormone present in the preparations according to the invention is selected from 3,3⁻,5-triiodo-L-thyronine and the pharmaceutically acceptable derivatives or precursors thereof. The thyroid hormone may thus be thyroxine, which exerts most, if not all, of its biological activity by conversion in the body to 3,3⁻,5-triiodo-L-thyronine.

The pharmaceutical compositions according to the invention are preferably formulated as creams, gels, ointments, pastes or lotions for topical application. For this purpose, any suitable carrier system normally employed in the preparation of such pharmaceutical compositions may be employed. For instance, the ingredients may be dispersed in an ointment base such as "unguentum merck" or dissolved in a lotion base such as aqueous ethanol. Systemic treatment using the oral or parenteral routes is also possible.

The compositions provide a concentration of anti-androgen of above 0.01 percent by weight and a concentration of thyroid hormone suitable to provide a concentration of at least 0.66 µg/ml. 3 ml is a typical single dose.

More preferably, the compositions may provide from 0.05—0.4% by weight anti-androgen, most preferably about 0.1% of each of an oestrogenic anti-androgen such as oestradiol benzoate and a non-oestrogenic antiandrogen such as medroxyprogesterone acetate.

2

Such a formulation is suitably applied about twice a day to the areas in which it is desired to prevent or arrest scalp hair loss and/or promote scalp hair growth.

The effectiveness of the compositions of the present invention may be enhanced by including therein other substances which are known to promote or suspected of being able to promote scalp hair growth. Examples of such substances are vasodilators, alpha-adrenergic blockers, anabolic growth-promoting agents, and "second messenger" hormone effector enzymes.

The following examples illustrate the present invention, and tentative conclusions have been drawn from the results so far achieved. It must be recognised, however, that, although for the most part human beings react in similar ways to the same drugs, individuals vary considerably as to their sensitivity to a particular drug. Hence the lower limits suggested herebelow for the effective levels of the components must be regarded with caution.

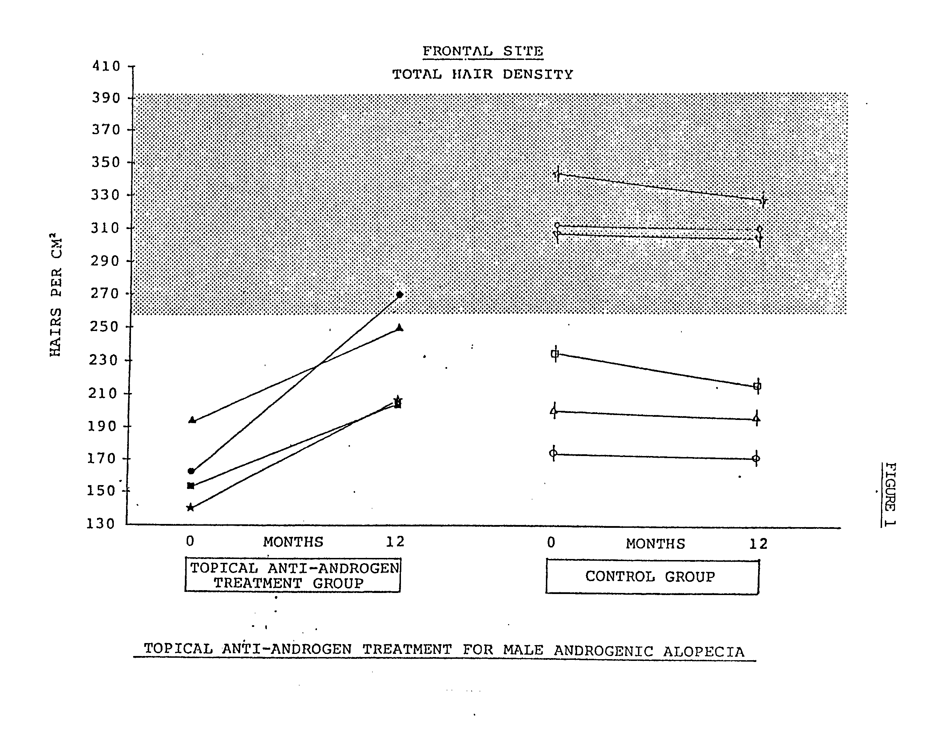

Clinical studies
1. Effect of a topical antiandrogen preparation on hair growth in four normal men compared with six untreated (control) men over one year (Trichogen I—Table 1; Figures 1, 2, 3 and 4)

Clinical studies using the application of a topical antiandrogen preparation were carried out in male patients and the response in hair growth compared with an untreated control group using the technique of the Unit Area Trichogram as previously described (The Unit Area Trichogram in the Assessment of Androgen-dependent Alopecia. Rushton H, James K. C., Mortimer C. H. *British Journal of Dermatology*: (1983), *109*, 429—437).

Four male patients aged between 27 years to 54 years with a history of common baldness of 6 to 24 years duration had Unit Area Trichograms carried out before and after 12 months of applying a topical antiandrogen preparation comprising:

Trichogen I
Oestradiol benzoate 0.1%
Medroxyprogesterone acetate 0.1%
3,3⁻,5-Triiodo-L-thyronine free acid 20 μg per 3 ml of solution

The preparation, total volume 3 ml, was applied twice daily to several sites in divided quantities to the frontal and vertex areas of the scalp.

Six male volunteers aged between 21 to 34 years with a history of common baldness of 1½ to 6 years duration formed the control group and did not receive any treatment.

Unit Area Trichograms in the occipital area were performed in 3 of the treatment group and 5 of the control group. The shaded area in the Figures identifies the normal range for the measurements shown and the individual symbols correspond to the same subjects, set out in Table 1.

All four men in the treatment group had an increase in *frontal total hair density* (hairs/cm²) during 12 months of therapy. None of the men in the control group had an increase in frontal total hair density during 12 months (Figure 1).

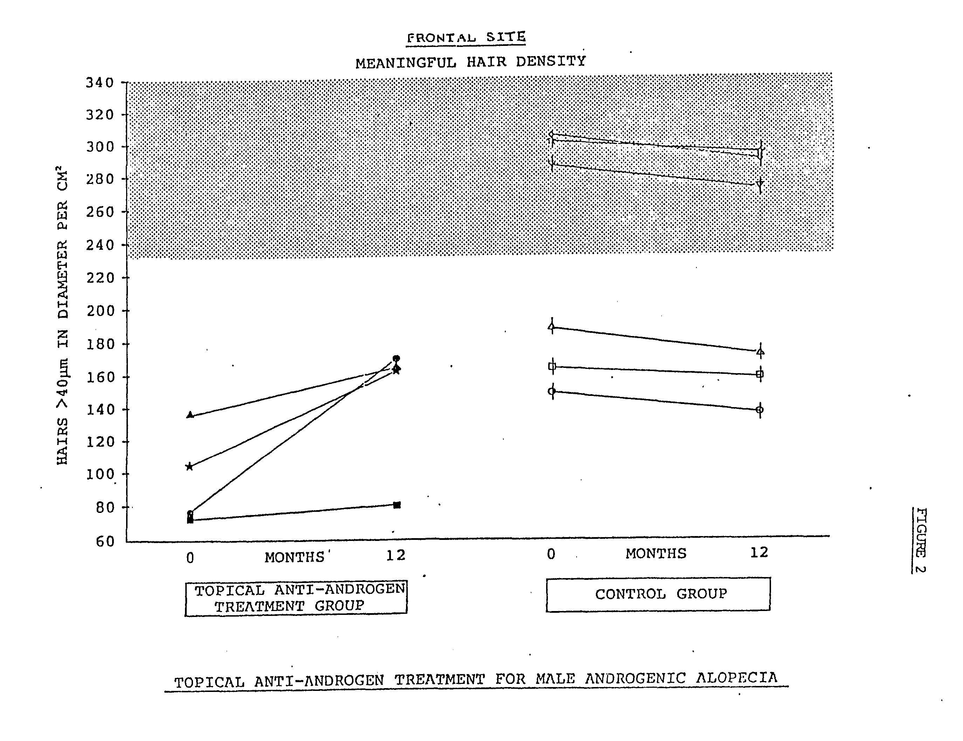

Three of the treated men showed an increase in *frontal meaningful hair density* (number of hairs greater than 40 μm in diameter/cm²; it is these hairs which have the capability of growing to full length) during 12 months of therapy. In one patient the increase in frontal meaningful density was not significant. No man in the control group showed an increase in meaningful hair density during 12 months (Figure 2).

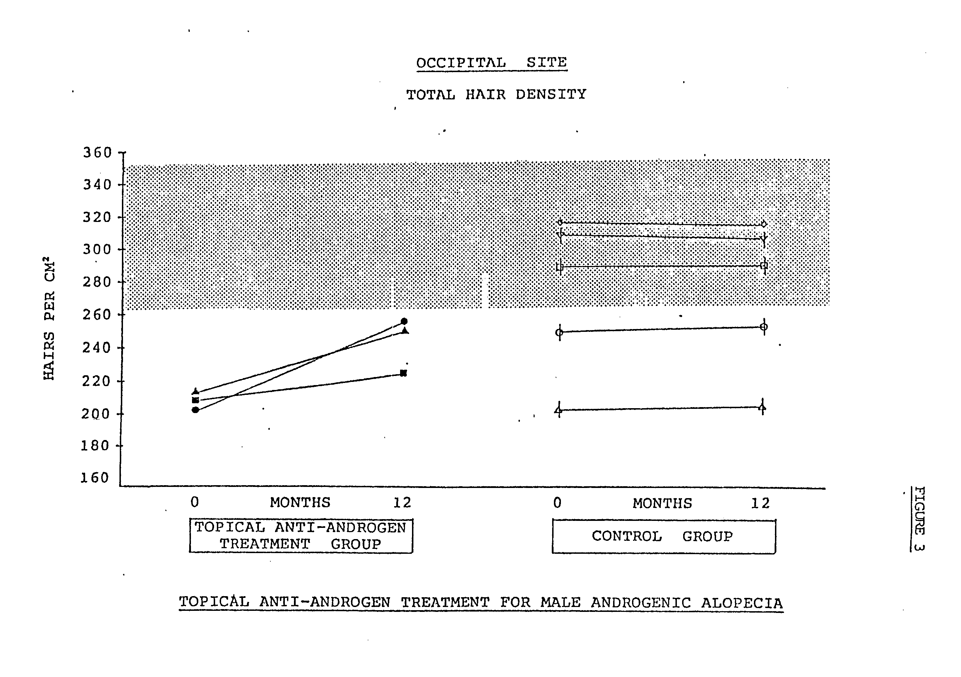

In each of the three treated men in whom *occipital total hair density* was measured there was an increase during 12 months of treatment. None of the five men in whom occipital total hair density was measured in the control group showed an increase in occipital total hair density during 12 months (Figure 3).

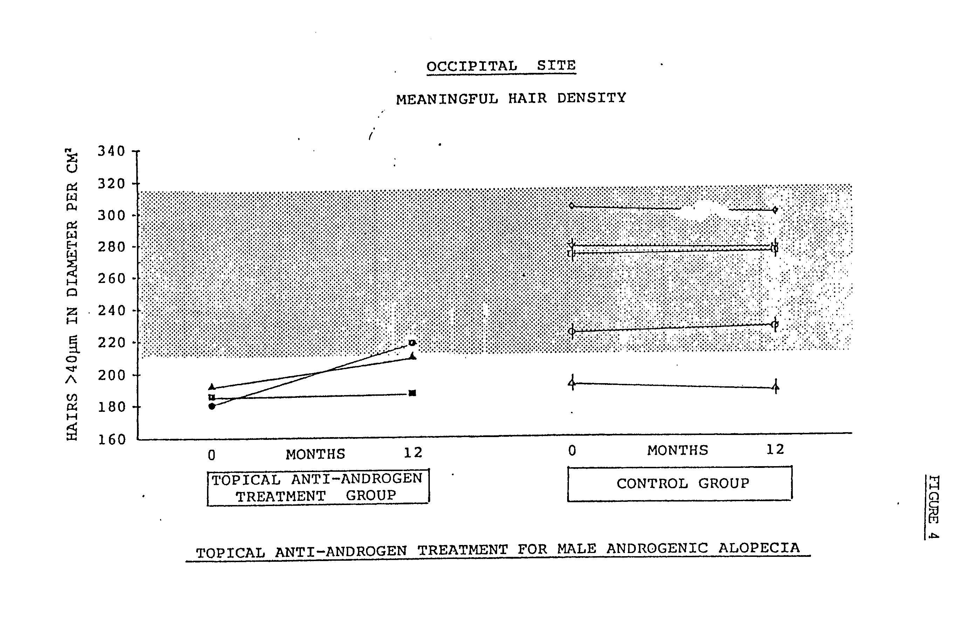

Two of the three men in the treatment group showed an increase in *occipital meaningful hair density* (number of hairs greater than 40 μm in diameter/cm²; it is these hairs which have the capability of growing to full length) during 12 months of therapy whereas one patient remained unchanged. None of the five men in the control group showed an increased in occipital meaningful hair density during 12 months (Figure 4).

Conclusion
The application of the above preparation Trichogen I, results in the arrest of the balding process with an increase in total hair density and meaningful hair density in both the frontal and occipital areas during 12 months of treatment. It is evident that there is cross-circulation with the effective dispersal of local antiandrogen activity between the frontal and occipital areas.

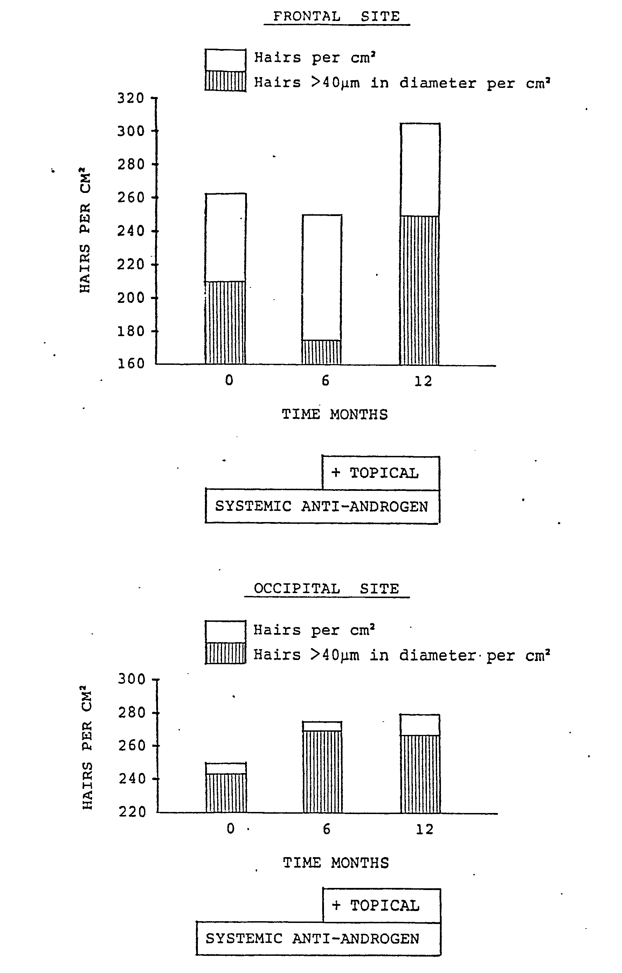

2. Effect of a topical antiandrogen preparation plus topical arterial vasodilator on hair growth in a single patient (Trichogen II—Figure 5)

A male patient aged 24 years with hair fall of 2 years duration with a clinical picture of male pattern baldness had the following results:

**0 123 528**

Before treatment:

Hormonal profile:

| | | |
|---|---|---|
| Testosterone | 20.8 nmol/l | (n 10—32) |
| SHBG | 31.0 nmol/l | (n 18—40) |
| Oestradiol | 125.0 pmol/l | (n 50—180) |
| Dihydrotestosterone | 1.5 nmol/l | (n 0.7—2.7) |

Semen analysis

| | | |
|---|---|---|
| *Total count | 8 millions | (n greater than 80 millions) |
| *Motility | 50% | (n greater than 60%) |
| Abnormal forms | 10% | (n up to 25%) |

The patient had previously had an undescended testis replaced in the scrotum (orchidopexy) in 1969. This condition is frequently associated with a reduced sperm count but otherwise normal testicular function with respect to testosterone secretion and potency.

| Unit area trichogram: | Frontal | Occipital |
|---|---|---|
| Total hair density (hairs/cm$^2$) | 263 (n 256—393) | 251 (n 261—356) |
| Meaningful hair density (hairs greater than 40 μm in diameter/cm$^2$) | 215 (n 232—340) | 246 (n 213—317) |

After 6 months of systemic antiandrogen treatment

Initially he began treatment with medroxyprogesterone acetate starting with 5 mg daily increasing to 40 mg daily at 6 months.

At this time the following hormone results were obtained:

| | | |
|---|---|---|
| Testosterone | 27.0 nmol/l | (n 10—32) |
| SHBG | 36.0 nmol/l | (n 18—40) |
| Oestradiol | 171.0 pmol/l | (n 50—180) |
| Dihydrotestosterone | 2.1 nmol/l | (n 0.7—2.7) |

Semen analysis

| | | |
|---|---|---|
| *Total count | 5 millions | (n greater than 80 millions) |
| *Motility | 10% | (n greater than 60%) |
| *Abnormal forms | 35% | (n up to 25%) |

Despite receiving medroxyprogesterone acetate orally, the patients plasma testosterone level was not suppressed and the Unit Area Trichogram was as follows:

| Unit area trichogram: | Frontal | Occipital |
|---|---|---|
| Total hair density (hairs/cm$^2$) | 248 (n 256—393) | 280 (n 261—356) |
| Meaningful hair density (hairs greater than 40 μm in diameter/cm$^2$) | 179 (n 232—340) | 271 (n 213—317) |

i.e. Worse in the frontal area.

The following measurements were then carried out:

| | | |
|---|---|---|
| *Dehydroepiandrosterone sulphate (DHEAS) | 9.4 μmol/l | (n 5—9) |
| *17 Alpha hydroxyprogesterone | 10.1 nmol/l | (n 0.6—6.0) |
| Androstenedione | 4.9 nmol/l | (n 3.1—10.0) |

4

**0 123 528**

These results indicated that the patient had not significantly suppressed total testosterone secretion while on medroxyprogesterone acetate and the elevated DHEAS and 17 alpha hydroxyprogesterone levels were consistent with an adrenal origin for the hormones. There had been a deterioration in the patients Unit Area Trichogram in the frontal area. At this stage it was decided to maintain the patients medroxyprogesterone acetate intake but to add topical antiandrogen therapy as:

Trichogen II
Medroxyprogesterone acetate 0.2%
Oestradiol benzoate 0.2%
3,3⁻,5-Triiodo-L-thyronine free acid in super-saturated solution
3,3⁻,5-Triiodo-D-thyronine free acid in super-saturated solution
Phentolamine mesylate 0.1% (as Rogitine, Ciba)
The patient applied 1 ml of the above preparation twice daily to the scalp.

After 6 months of the same dose of systemic medroxyprogesterone acetate plus Trichogen II the following results were obtained:

| | | |
|---|---|---|
| Testosterone | 31.0 nmol/l | (n 10—32) |
| SHBG | 19.0 nmol/l | (n 18—40) |
| Oestradiol | 125.0 pmol/l | (n 50—180) |
| Dihydrotestosterone | 2.1 nmol/l | (n 0.7—2.7) |
| *DHEAS | 10.0 µmol/l | (n 5—9) |
| *17 Hydroxyprogesterone | 6.7 nmol/l | (n 0.6—6.0) |
| Androstenedione | 5.8 nmol/l | (n 3.1—10.0) |

Semen analysis

| | | |
|---|---|---|
| *Total count | 16.8 millions | (n greater than 80 millions) |
| Motility | 65% | (n greater than 60%) |
| Abnormal forms | 10% | (n up to 25%) |

The Unit Area Trichogram showed:

| Unit area trichogram: | Frontal | Occipital |
|---|---|---|
| Total hair density (hairs/cm²) | 307 (n 256—393) | 282 (n 261—356) |
| Meaningful hair density (hairs greater than 40 µm in diameter/cm²) | 248 (n 232—340) | 263 (n 213—317) |

The above Unit Area Trichogram results are shown in diagrammatic form (Figure 5).

The above results show that in this patient with male pattern baldness medroxyprogesterone acetate up to 40 mg per day while being effective in most men to suppress pituitary LH and FSH secretion was not effective in reducing this patients circulating testosterone levels since in addition to androgens of testicular origin there was evidence of an adrenal source for these. During the first 6 months of systemic medroxyprogesterone acetate treatment there was no significant reduction in circulating androgen levels and the patients frontal hair density studies showed a deterioration. There was an improvement in the occipital region. This is generally to be expected since this area is more resistant to the balding process and recovers more quickly than the frontal area. It is probable that the inhibition of 5 alpha reductase activity with decreased conversion of testosterone to dihydrotestosterone in the scalp of the occipital area was sufficient to allow new hair growth without suppression of circulating androgen levels.

After a further 6 months of systemic medroxyprogesterone acetate treatment circulating androgen levels were still not reduced and in fact higher than the basal levels. However, during this time, the addition of Trichogen II resulted in new hair growth in the frontal area. This must have been due to the local inhibition of androgen activity in the scalp by local absorption of Trichogen II.

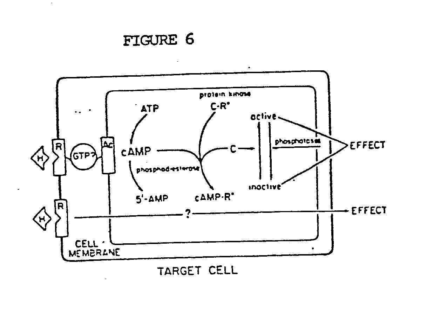

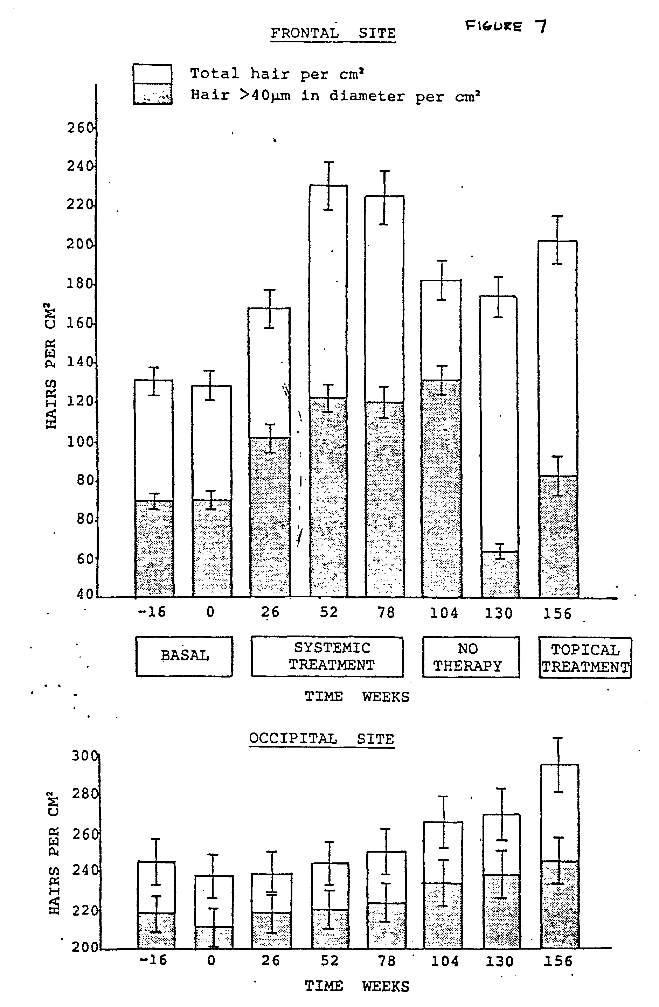

3. Effect of a topical antiandrogen preparation plus cyclic AMP/ATP/phosphodiesterase inhibitor on hair growth in a single male patient (Trichogen III)—Figures 6, 7

It is recognised that many hormones including steroids act by the activation of the cyclic AMP

5

mechanism. In this process hormones interact with specific receptors on the cell membrane. This results in the activation of membrane bound adenylate cyclase which enhances the conversion of ATP (adenosine triphosphate) to cyclic AMP (cyclic adenosine 3,5, monophosphate). Cyclic AMP is known as the ubiquitous second messenger since it is able to relay information derived from numerous substances binding at the cell surface. The cyclic AMP then activates a variety of protein kinases by combining with the regulatory sub-group of the enzyme which is then dissociated from the catalytic sub-group. The catalytic sub-unit then catalyses the phosphorylation of certain key proteins, usually enzymes, which then leads to the action of the original hormone which presented at the cell membrane. This mechanism also results in the elaboration of various proteins which may themselves be hormones or comprise specific tissue e.g. muscle tissue, nerve tissue, hair. The process may also be modified by the availability of calcium, cyclic GMP (cyclic guanosine 3,5-monophosphate) or other enzyme systems. Other hormones may achieve their action by entering the cell directly to bind with specific cytoplasmic receptors which are then modified and transport the hormone receptor complex into the nucleus where specific genes are activated to produce the end result. These mechanisms are shown diagrammatically in Figure 6. Cyclic AMP is inactivated by phosphodiesterase. The latter enzyme itself can be inactivated by theophylline and caffeine thereby leading to an increase in the cyclic AMP concentration in certain tissues.

In order to enhance the effects of the topical antiandrogen preparation a further solution was made up to contain:

Trichogen III

Oestradiol benzoate 0.2%

Medroxyprogesterone acetate 0.2%

3,3⁻,5-Triiodo-L-thyronine free acid in super-saturated solution

Phentolamine mesylate 0.04%

Cyclic AMP free acid 0.1%

Cyclic AMP sodium 0.05%

ATP Magnesium 0.1%

Choline theophyllinate 0.05%

A 25 year old man with common baldness had previously been treated with medroxyprogesterone acetate increasing to 40 mg daily until 68 weeks when the dose was reduced to 20 mg daily, then after 72 weeks to 10 mg daily until 78 weeks when treatment was stopped. The results of the Unit Area Trichogram are shown in Figure 7. After 26 weeks without systemic medroxyprogesterone acetate the patient showed a decrease in total hair density in the frontal site. After 52 weeks without treatment there was also a marked decrease in meaningful hair density in the frontal site. The occipital region remained unchanged. Instead of re-introducing systemic medroxyprogesterone acetate the patient began treatment with the above preparation 2 ml applied 4 times daily to the scalp in the frontal and vertex areas.

After 26 weeks of topical treatment with Trichogen III there was an increase in total hair density in the frontal site from 179 hairs/cm² to 203 hairs/cm². There was also an increase in frontal meaningful hair density from 64 hairs/cm² to 93 hairs/cm². In the occipital region there was an increase in total hair density from 267 hairs/cm² to 293 hairs/cm² while meaningful hair density was not significantly changed from 239 hairs/cm² to 246 hairs/cm².

The error bars of the trichogram technique are shown in the figure for each measurement.

Conclusion

The topical application of Trichogen III resulted in significantly increased total hair density and meaningful hair density in the frontal region. It is proposed that the addition of cyclic AMP, ATP and choline theophyllinate will enhance the anti-androgenic action of Trichogen I and II.

4. Ineffective topical preparations applied to the scalp

The following preparations were found to be ineffective in increasing hair density in common baldness in men:

a) Oestradiol benzoate 0.01% Medroxyprogesterone acetate 0.01% 3,3⁻,5-Triiodo-L-thyronine free acid 20 µg/3 ml

b) Oestradiol benzoate 0.1% 3,3⁻,5-Triiodo-L-thyronine free acid 20 µg/3 ml

c) Oestradiol benzoate 0.1%

Medroxyprogesterone acetate 0.1%

d) Oestradiol benzoate 0.1%

e) Oestradiol benzoate 0.01%

f) Medroxyprogesterone acetate 0.1%

g) Medroxyprogesterone acetate 0.01%

h) 3,3⁻,5-Triiodo-L-thyronine free acid 20 µg/3 ml

i) 3,3⁻,5-Triiodo-D-thyronine free acid was not an effective substitute in any concentration for the -L-form

j) Progesterone 0.1%

k) Progesterone 0.01%.

# 0 123 528

None of the men who received topical treatment had any systemic side effects. All retained normal potency and beard growth. Three men who received the highest concentration of individual hormonal constituents (oestradiol benzoate 0.2%, medroxyprogesterone acetate 0.2%, 3,3⁻,5-triiodo-L-thyronine free acid super-saturated solution) had full haematology, liver function tests, urea and electrolytes, endocrine profile and sperm counts carried out before, after 3 months and after 6 months of applying the preparation 2 ml twice daily to the scalp without any significant change in the parameters measured. The patient who received this maximum concentration plus cyclic AMP free acid 0.1%; cyclic AMP sodium 0.05%, ATP magnesium 0.1%, phentolamine 0.04% and choline theophyllinate 0.05% also had no change in potency nor beard growth and without any significant change in laboratory investigations and sperm count when the same parameters were checked before, after 3 months and after 6 months of applying the preparation, 2 ml 4 times daily to the scalp. All patients noted a decrease in the greasiness of the hair and improved "condition" and cosmetic appearance. Three patients who had experienced superficial scalp tenderness and irritation prior to treatment noted the rapid alleviation of these symptoms within 1—2 days of beginning topical treatment and for its duration.

5. Optimum formulation of preparation

From the above it is evident that oestradiol benzoate 0.1% alone or medroxyprogesterone acetate 0.1% alone or 3,3⁻,5-triiodo-L-thyronine 20 µg in 3 ml of solution alone is not effective in producing hair growth in common baldness. Combinations of any two of the three are also not effective. 3,3⁻,5-triiodo-D-thyronine free acid is not an effective substitute for the -L- form.

It appears necessary to combine oestradiol benzoate, medroxyprogesterone acetate with the triiodothyronine to achieve therapeutic effect.

When the above combination was used but in reduced doses as oestradiol benzoate 0.01%, medroxyprogesterone acetate 0.01% while maintaining triiodothyronine at 20 µg/3 ml there was no effect.

Thus:

1. The minimum effective dose of oestradiol benzoate and medroxyprogesterone acetate is between 0.01% and 0.1% when combined with triiodothyronine at 20 µg/3 ml application.

The concentration of the main ingredients could be increased to oestradiol benzoate 0.2%, medroxyprogesterone acetate 0.2% and triiodothyronine to the point of producing a super-saturated solution without causing local scalp irritation or producing systemic side effects. The solubility of these compounds in the solvent solution was maximal at room temperature.

Thus:

2. There would appear to be no added advantage in increasing the concentrations still further since it might be expected that the constituents would crystallise out on the scalp, becoming a powder.

Having regard for the known mechanism of action of steroids (and probably other hormones) cyclic AMP, ATP and other normally intra-cellular enzymes e.g. cyclic GMP could be added to the formulation without impairing the effect or causing side effects and may produce added benefit in certain subjects. The concentrations of cyclic AMP and ATP were also maximal to produce a super-saturated solution at room temperature.

It was possible to add a vasodilator preparation, phentolamine mesylate to a concentration of 0.04%—0.1% and probably higher. This preparation is a well recognised alpha-adrenergic blocker which may be expected to cause dilatation of peripheral capillaries in the scalp thereby increasing the blood supply to the hair follicle. When similar preparations are applied in solution in propylene glycol, industrial methylated spirit and water or in Ungentum Merck at 1% concentration as minoxidil, hair growth can be achieved in patients with alopecia areata (see topical minoxidil in the Treatment of Alopecia Areata. Fenton D. A., Wilkinson J. D. *British Medical Journal: 287*, 1015—1017).

I propose that the combination of the topical antiandrogen preparation with an alpha-adrenergic receptor blocker or other vasodilator may enhance scalp hair growth in common baldness and also in alopecia areata by diminishing any additional inhibitory effects on hair growth by the common baldness process coexisting with alopecia areata. Three female patients with alopecia areata progressing to alopecia totalis capitis whom I have treated previously with cyclical antiandrogen therapy as:

Cyproterone acetate 50 mg daily for the first 10 days of each cycle. Ethinyloestradiol 40 µg daily for 21 days of each cycle showed within 2—4 months the growth of fine downy hair over the scalp. Subsequently, this fell only to regrow again in six months. One patient had continued with treatment for 18 months and was growing terminal hair. Therefore, suppression of androgenic effects of endogenous hormones may potentiate the response to vasodilator substances.

Thus:

3. The combination of topical antiandrogen therapy with topical alpha-adrenergic receptor blocker or other vasodilator may produce an additional beneficial effect on scalp hair growth in common baldness as well as alopecia areata/totalis/universalis.

4. The combination of topical antiandrogen therapy as described with other known antiandrogens (e.g. cimetadine, azelaic acid) given topically or systemically, or with anabolic growth promoting substances e.g. growth hormone, may produce an additional beneficial effect on scalp hair growth in common baldness.

5. Topical antiandrogen therapy may be expected to produce increased hair growth (rate and/or density and/or fibre thickness) in apparently normal subjects and may be useful in the treatment of other conditions in which hair loss occurs, e.g. following general viral, bacterial illness, post-pregnancy, pre-, peri-, post-menopausal women, post-irradiation; post-cytotoxic therapy, malnutrition, parasitic infections, metabolic disorders, infective conditions, following burns, physical or chemical injury to the scalp or in other conditions in which hair loss occurs.

6. Topical antiandrogen therapy may be used in patients with greasy hair and irritating scalp conditions and to improve the condition and appearance of existing hair.

7. Topical antiandrogen therapy may be in used in both male and female patients.

TABLE 1

Comparison between treatment group and control group in the treatment of male androgenic alopecia, following topical anti-androgen therapy

| Treatment group: Frontal site | | | | | Occipital site | | | |
|---|---|---|---|---|---|---|---|---|
| | ℙ THD | | § MHD | | ℙ THD | | § MHD | |
| Time Months | 0 | 12 | 0 | 12 | 0 | 12 | 0 | 12 |
| ▲ P  S | 195 | 250 | 139 | 163 | 212 | 241 | 194 | 214 |
| ● J  M | 166 | 270 | 74 | 166 | 203 | 255 | 181 | 220 |
| ■ E  D | 155 | 205 | 74 | 79 | 210 | 222 | 192 | 192 |
| * A  L | 137 | 206 | 108 | 162 | | | | |

| Control group: Frontal site | | | | | Occipital site | | | |
|---|---|---|---|---|---|---|---|---|
| | ℙ THD | | § MHD | | ℙ THD | | § MHD | |
| Time Months | 0 | 12 | 0 | 12 | 0 | 12 | 0 | 12 |
| ⸸ R  H | 346 | 330 | 302 | 297 | | | | |
| ◇ A  C | 313 | 308 | 305 | 295 | 310 | 306 | 303 | 297 |
| ▽ T  B | 310 | 303 | 285 | 276 | 305 | 300 | 273 | 270 |
| ⊡ G  H | 235 | 216 | 165 | 159 | 289 | 286 | 272 | 270 |
| △ J  S | 209 | 205 | 184 | 174 | 200 | 206 | 192 | 188 |
| ① F  A | 176 | 173 | 150 | 137 | 246 | 250 | 223 | 234 |

ℙ THD—Hairs per $cm^2$
§ MHD—Hairs >40 μm in diameter per $cm^2$.

The above results suggest that it is the combination of the three essential active agents, viz. a thyroid hormone, an oestrogen and a non-oestrogenic anti-androgen, acting together in the region of the hair follicles that promotes hair growth. It will be appreciated, therefore, that it is not necessary to administer all three agents by the same route. Thus, for example, the thyroid hormone could be taken orally as a tablet, whilst the oestrogen and the non-oestrogenic anti-androgen could be applied topically as a lotion. Clearly, however, the oestrogen would normally not be administered to a male patient via the systemic route, because of adverse side effects.

Accordingly the present invention further provides a pack for a combination of pharmaceutical compositions for promoting scalp hair growth, which pack comprises at least one composition for systemic application and at least one composition for topical application, wherein each composition contains individually as ingredients a thyroid hormone, an oestrogen, a non-oestrogenic anti-androgen, or a mixture thereof, with the proviso that all three ingredients are present within the pack.

Also within the scope of the invention is the use of a combination of topical and systemic pharmaceutical compositions for promoting scalp hair growth wherein the combination includes as ingredients a thyroid hormone, an oestrogen and a non-oestrogenic anti-androgen.

8

**Claims**

1. A pharmaceutical composition for promoting scalp hair growth comprising in combination an oestrogen in a concentration above 0.01% by weight to the maximum solubility of same in its solvent, a non-oestrogenic anti-androgen in a concentration above 0.01% by weight to the maximum solubility of same in its solvent and an amount of thyroid hormone selected from 3,3⁻,5-triiodo-L-thyronine and the pharmaceutically acceptable derivatives or precursors thereof, sufficient to provide a concentration of at least 0.66 µg/ml to the maximum solubility of same in its solvent.

2. A pharmaceutical composition as claimed in claim 1 wherein the thyroid hormone is thyroxine or triiodothyronine.

3. A pharmaceutical composition as claimed in claim 1 or claim 2 comprising as the anti-androgen a pharmaceutically acceptable derivative of medroxyprogesterone or cyproterone.

4. A pharmaceutical composition as claimed in claim 3 comprising as the anti-androgen medroxyprogesterone acetate or cyproterone acetate.

5. A pharmaceutical composition as claimed in any one of the preceding claims wherein the oestrogen is oestradiol or a pharmaceutically acceptable derivative thereof.

6. A pharmaceutical composition as claimed in any one of the preceding claims including at least one vasodilator, alpha-adrenergic blocker, anabolic growth-promoting agent, or "second messenger" hormone effector enzyme.

7. A pharmaceutical composition as claimed in any one of the preceding claims in the form of a cream, gel, ointment, paste, or lotion for topical application.

8. A pharmaceutical composition as claimed in any one of the preceding claims in a form for oral or parenteral administration.

9. A pack for a combination of pharmaceutical compositions for promoting scalp hair growth, which pack comprises at least one composition for systemic application and at least one composition for topical application, wherein each composition contains individually as ingredients a thyroid hormone, an oestrogen, a non-oestrogenic anti-androgen, or a mixture thereof, with the proviso that all three ingredients are present within the pack.

**Patentansprüche**

1. Pharmazeutische Verbindung für das Fördern des Haupthaarwachstums, die in Kombination ein Östrogen in einer Konzentration von mehr als 0,01 Gew.-% bis zur maximalen Löslichkeit des Östrogens in seinem Lösungsmittel, ein nicht-östrogenisches Antiandrogen in einer Konzentration von mehr als 0,01 Gew.-% bis zur maximalen Löslichkeit des Antiandrogens in seinem Lösungsmittel und eine Menge Thyroïd- bzw. Schilddrüsenhormon, das aus 3,3⁻,5-Trijod-L-Thyronin und dessen pharmazeutisch verträglichen Derivaten oder Vorläuferprodukten besteht, enthält, welche Menge ausreicht, um eine Konzentration von mindestens 0,66 µg/ml bis zur maximalen Löslichkeit desselben in seinem Lösungsmittel zu schaffen.

2. Pharmazeutische Verbindung nach Anspruch 1, dadurch gekennzeichnet, daß das Schilddrüsenhormon Thyroxin oder Trijodthyronin ist.

3. Pharmazeutische Verbindung nach Anspruch 1 oder 2, gekennzeichnet durch ein pharmazeutisch verträgliches Derivat von Medroxyprogesteron oder Cyproteron als Antiandrogen.

4. Pharmazeutische Verbindung nach Anspruch 3, gekennzeichnet durch das Medroxyprogesteronacetat oder Cyproteronacetat als Antiandrogen.

5. Pharmazeutische Verbindung nach einem der vorhergehenden Ansprüche, dadurch gekennzeichnet, daß das Östrogen Östradiol oder ein pharmazeutisch verträgliches Derivat von Östradiol ist.

6. Pharmazeutische Verbindung nach einem der vorhergehenden Ansprüche, dadurch gekennzeichnet, daß mindestens ein Vasodilator, ein alpha-adrinergischer Blocker, ein wachstumsförderndes Anabolikum oder ein "second messenger"-Hormon-Auslöser-Enzym vorgesehen ist.

7. Pharmazeutische Verbindung nach einem der vorhergehenden Ansprüche, in Form von Creme, Gel, Salbe, Paste oder in Form einer Lotion für örtliche Anwendung.

8. Pharmazeutische Verbindung nach einem der vorhergehenden Ansprüche, in einer für orale oder parenterale Verabreichung geeigneten Form.

9. Packung für eine Kombination von pharmazeutischen Verbindungen für das Fördern des Haupthaarwachstums, welche Packung mindestens eine Verbindung für systemische Anwendung und mindestens eine Verbindung für örtliche Anwendung aufweist, wobei jede Verbindung als Bestandteil ein Schilddrüsenhormon, ein Östrogen, ein nichtöstrogenisches Antiandrogen oder eine Mischung dieser mit der Maßgabe aufweist, daß alle drei Bestandteile in der Packung vorhanden sind.

**Revendications**

1. Composition pharmaceutique destinée à favoriser la pousse des cheveux, comprenant, en combinaision, un oestrogène à une concentration supérieure à 0,01% en poids jusqu'à la solubilité maximale de celui-ci dans son solvant, un anti-androgène non oestrogénique à une concentration supérieure à 0,01% en poids jusqu'à la solubilité maximale de ce dernier dans son solvant, et une quantité d'une hormone thyroïdienne choisie parmi la triiodo-3,3⁻,5-L-thyronine et ses dérivés ou précurseurs pharmaceutiquement acceptables, qui est suffisante pour fournir une concentration d'au moins 0,66 μg/ml jusqu'à la solubilité maximale de celle-ci dans son solvant.

2. Composition pharmaceutique selon la revendication 1, caractérisée en ce que l'hormone thyroïdienne est la thyroxine ou la triiodothyronine.

3. Composition pharmaceutique selon la revendication 1 ou la revendication 2, caractérisée en ce qu'elle comprend, comme anti-androgène, un dérivé pharmaceutiquement acceptable de la médroxyprogestérone ou de la cyprotérone.

4. Composition pharmaceutique selon la revendication 3, caractérisée en ce qu'elle comprend, comme anti-androgène, l'acétate de médroxyprogestérone ou l'acétate de cyprotérone.

5. Composition pharmaceutique selon l'une des revendications précédentes, caractérisée en ce que l'oestrogène est l'oestradiol ou un dérivé pharmaceutiquement acceptable de celui-ci.

6. Composition pharmaceutique selon l'une des revendications précédentes, caractérisée en ce qu'elle comprend au moins un vasodilatateur, un adrénolytique alphabloquant, un agent anabolique favorisant la croissance, ou une enzyme effectrice d'hormone "second messager".

7. Composition pharmaceutique selon l'une des revendications précédentes, caractérisée en ce qu'elle se présente sous la forme d'une crème, d'un gel, d'une pommade, d'une pâte, ou d'une lotion pour l'application topique.

8. Composition pharmaceutique selon l'une des revendications précédentes, caractérisée en ce qu'elle se présente sous une forme appropriée pour l'administration orale ou parentérale.

9. Coffret pour une combinaison de compositions pharmaceutiques destinées à favoriser la pousse des cheveux, ledit coffret comprenant au moins une composition pour l'application systémique et au moins une composition pour l'application topique, suivant lequel chaque composition contient individuellement, en tant qu'ingrédients, une hormone thyroïdienne, un oestrogène, un anti-androgène non oestrogénique, ou un mélange de ces ingrédients, à la condition que la totalité des trois ingrédients soient présents à l'intérieur du coffret.

FRONTAL SITE
TOTAL HAIR DENSITY

TOPICAL ANTI-ANDROGEN TREATMENT FOR MALE ANDROGENIC ALOPECIA

FIGURE 1

0 123 528

FRONTAL SITE
MEANINGFUL HAIR DENSITY

TOPICAL ANTI-ANDROGEN TREATMENT FOR MALE ANDROGENIC ALOPECIA

FIGURE 2

0 123 528

FIGURE 3

OCCIPITAL SITE

TOTAL HAIR DENSITY

HAIRS PER CM²

360
340
320
300
280
260
240
220
200
180
160

MONTHS
0    12
TOPICAL ANTI-ANDROGEN TREATMENT GROUP

MONTHS
0    12
CONTROL GROUP

TOPICAL ANTI-ANDROGEN TREATMENT FOR MALE ANDROGENIC ALOPECIA

3

OCCIPITAL SITE

MEANINGFUL HAIR DENSITY

TOPICAL ANTI-ANDROGEN TREATMENT FOR MALE ANDROGENIC ALOPECIA

0 123 528

FIGURE 4

FRONTAL   SITE

☐ Hairs per cm²
▥ Hairs >40μm in diameter per cm²

TIME MONTHS

+ TOPICAL
SYSTEMIC ANTI-ANDROGEN

OCCIPITAL   SITE

☐ Hairs per cm²
▥ Hairs >40μm in diameter·per cm²

TIME MONTHS

+ TOPICAL
SYSTEMIC ANTI-ANDROGEN

5

FIGURE 6

TARGET CELL

FRONTAL SITE    FIGURE 7

Total hair per cm²
Hair >40μm in diameter per cm²

FRONTAL SITE chart — HAIRS PER CM² vs TIME WEEKS (-16, 0, 26, 52, 78, 104, 130, 156). Treatment groups: BASAL, SYSTEMIC TREATMENT, NO THERAPY, TOPICAL TREATMENT.

OCCIPITAL SITE

OCCIPITAL SITE chart — HAIRS PER CM² vs TIME WEEKS (-16, 0, 26, 52, 78, 104, 130, 156).

7